# EUROPEAN PATENT SPECIFICATION

(11) **EP 0 675 102 B1**
(45) Date of publication and mention of the grant of the patent: **14.06.2000**
(21) Application number: 95104719.0
(22) Date of filing: 30.03.1995
(51) Int. Cl.: C07C 67/36, C07C 69/738, C07C 231/12, C07C 253/30

(54) **A process for producing a substituted benzoyl fatty acid derivative**
Verfahren zur Herstellung von substituierten Benzoylfettsäurederivaten
Procédé pour la préparation d'un dérivé substitué d'acide gras benzoyle

(30) Priority: 31.03.1994 JP 12948094
(43) Date of publication of application: 04.10.1995
(73) Proprietor: NIHON NOHYAKU CO., LTD., Chuo-ku Tokyo100-0027 (JP)
(72) Inventor: Yoshiura, Akihiko, Amagasaki-shi (JP); Abe, Noboru, Nishinomiya-shi (JP); Kudo, Masaaki, Shijonawate-shi (JP)
(74) Representative: Grünecker, Kinkeldey, Stockmair & Schwanhäusser Anwaltssozietät

(56) References cited:
- EP-A- 0 081 384
- J. ORG. CHEM., vol. 40, no. 4, 1975, pages 532-534, XP002007990 J. K. STILLE AND P. K. WONG: "Carbonylation of Aryl and Benzyl Halides Catalyzed by Dichlorobis(triphenylphosphine)palladium(I I)"
- SYNTHETIC COMMUNICATIONS, vol. 20, no. 17, 1990, pages 2631-2640, XP002022514 M. L. KANTAM ET AL.: "Cobalt catalysed carbonylation of organic halides under solid-liquid phase transfer conditions"

## Description

The present invention relates to a process for producing a substituted benzoyl fatty acid derivative represented by the general formula (I): 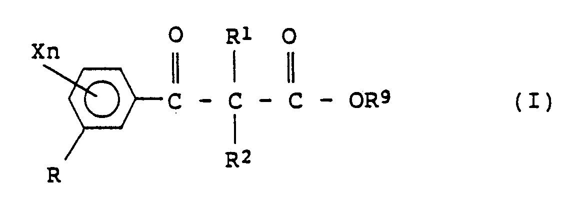 [wherein R is a hydrogen atom or

-O-C(R³)(R⁴)-R⁵

(wherein R³ and R⁴, which may be the same or different, are hydrogen atoms or C₁₋₆alkyl groups, and R⁵ is a cyano group,

-CON(R⁶)R⁷

(wherein R⁶ and R⁷, which may be the same or different, are hydrogen atoms or C₁₋₆alkyl groups) or

-COAR⁸

(wherein R⁸ is a hydrogen atom or a C₁₋₆alkyl group, and A is an oxygen atom or a sulfur atom)), each of R¹ and R² is a hydrogen atom or a C₁₋₆alkyl group, R⁹ is a C₁₋₆alkyl group, an unsubstituted phenyl group, or a substituted phenyl group having one or more substituents which may be the same or different and are selected from the group consisting of halogen atoms, C₁₋₆alkyl groups, halo-C₁₋₆alkyl groups, C₁₋₆alkoxy groups, halo-C₁₋₆alkoxy groups, C₁₋₆alkylthio groups and halo-C₁₋₆alkylthio groups, X's, which may be the same or different, are halogen atoms, and n is an integer of 1 to 3].

Substituted benzoyl fatty acid derivatives are compounds useful as intermediates of medicines, agrochemicals or chemical products.

J.O.C. 40(4), 1975, 532-534 and Synth., Commun., 20(17), 1990, 2631-2640 disclose processes for production of benzoyl fatty acids.

However, the catalysts used in the above-mentioned prior art references are expensive and hence economically disadvantageous.

It is the object of the present invention to provide a novel process for producing a substituted benzoyl fatty acid which can be practiced using more inexpensive materials and catalyst than those used in prior art, gives an improved yield, requires only a reduced amount of the catalyst, permits recovery of the catalyst, and is thus more economical.

This object has been achieved with a process for producing a substituted benzoyl fatty acid derivative represented by the general formula (I): 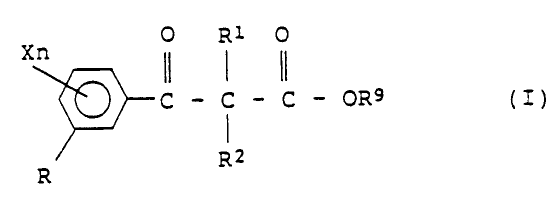 [wherein R is a hydrogen atom or

-O-C(R³)(R⁴)-R⁵

(wherein R³ and R⁴, which may be the same or different, are hydrogen atoms or C₁₋₆alkyl groups, and R⁵ is a cyano group,

-CON(R⁶)R⁷

(wherein R⁶ and R⁷, which may be the same or different, are hydrogen atoms or C₁₋₆alkyl groups) or

-COAR⁸

(wherein R⁸ is a hydrogen atom or a C₁₋₆alkyl group, and A is an oxygen atom or a sulfur atom)), each of R¹ and R² is a hydrogen atom or a C₁₋₆alkyl group, R⁹ is a C₁₋₆alkyl group, an unsubstituted phenyl group, or a substituted phenyl group having one or more substituents selected from the group consisting of halogen atoms, C₁₋₆alkyl groups, halo-C₁₋₆alkyl groups, C₁₋₆alkoxy groups, halo-C₁₋₆alkoxy groups, C₁₋₆alkylthio groups and halo-C₁₋₆alkylthio groups, X's, which may be the same or different, are halogen atoms, and n is an integer of 1 to 3] which comprises reacting a substituted phenacyl halide derivative represented by the general formula (II): 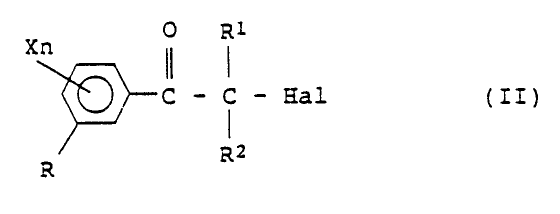 (wherein R, R¹, R², X and n are as defined above, and Hal is a chlorine atom) with a compound represented by the general formula (III):

R⁹-OH

(wherein R⁹ is as defined above) and carbon monoxide in the presence of a base, a palladium compound and at least one phosphorus compound.

In the definition of the substituted benzoyl fatty acid derivative of the general formula (I) according to the present invention, the term "C₁₋₆alkyl group" means a linear or branched alkyl group having 1 to 6 carbon atoms, such as methyl, ethyl, n-propyl, i-propyl, n-butyl, i-butyl, s-butyl, t-butyl, n-pentyl, n-hexyl or the like. The halogen atoms include chlorine atom, fluorine atom, iodine atom or bromine atom. For example, the term "haloalkyl group" means a substituted and linear or branched alkyl group of 1-6 carbon atoms having as the substituent(s) one or more halogen atoms which may be the same or different.

The production process of the present invention can be schematically shown, for example, as follows: 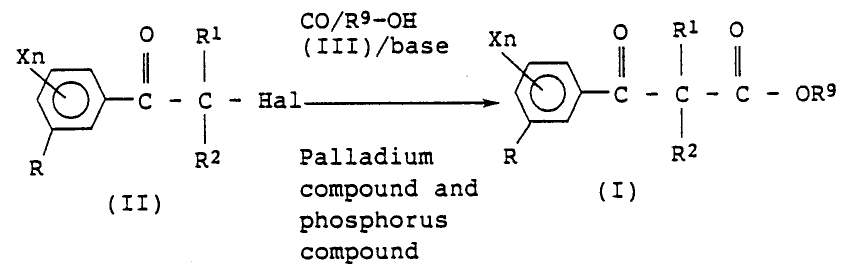 wherein R, R¹, R², R⁹, X, n and Hal are as defined above.

A substituted benzoyl fatty acid derivative of the general formula (I) can be produced by reacting a phenacyl halide derivative of the general formula (II) with carbon monoxide and a compound of the general formula (III) in the presence of a base and in the presence or absence of an inert solvent by using a combination of a palladium compound and at least one phosphorus compound as a catalyst.

It is sufficient that the palladium compound used as a catalyst in the present invention is used in combination with at least one phosphorus compound. As the palladium compound, there can be used, for example, metallic palladium or a zerovalent or divalent palladium complex supported on a solid. Examples include palladium-carbon, palladium-alumina, palladium chloride, palladium bromide, palladium acetate, dichlorobis(cyanophenyl)palladium, dichlorobis(triphenylphosphine)palladium, tetrakis(triphenylphosphine)palladium, dichlorobis(benzonitrile)palladium or dichlorobis(acetonitrile)palladium.

As the phosphorus compound(s) usable in combination with the palladium compound in the present invention, there can be exemplified trialkylphosphines (e.g. trimethylphosphine and triethylphosphine), triarylphosphines (e.g. triphenylphosphine), phosphites having three substituents which may be the same or different and are selected from the group consisting of alkyl groups and aryl groups (e.g. trimethyl phosphites, triethyl phosphites and triphenyl phosphites), and phosphorus compounds represented by the general formula (IV):

(R')₂-P-Y-P-(R')₂ (IV)

(wherein R' is a C_{1₋6}alkyl group, an unsubstituted phenyl group, or a substituted phenyl group having one or more substituents selected from the group consisting of halogen atoms, C₁₋₆alkyl groups, halo-C₁₋₆alkyl groups, C₁₋₆alkoxy groups, halo-C₁₋₆alkoxy groups, C₁₋₆alkylthio groups and halo-C₁₋₆alkylthio groups, and Y is a C₁₋₆alkylene group), for example, bis(dialkylphosphino)alkanes [e.g. 1,1-bis(dimethylphosphino)methane, 1,1-bis(diethylphosphino)methane, 1,2-bis(dimethylphosphino)ethane, 1,2-bis(diethylphosphino)ethane, 1,3-bis(dimethylphosphino)propane and 1,4-bis(dimethylphosphino)butane], and bis(diarylphosphino)alkanes [e.g. 1,1-bis(diphenylphosphino)methane, 1,2-bis(diphenylphosphino)ethane, 1,3-bis(diphenylphosphino)propane and 1,4-bis (diphenylphosphino)butane].

The amount of the phosphorus compound(s) used is 0.01 to 10,000 moles, preferably 0.1 to 100 moles, per mole of the palladium compound.

In the present invention, it is sufficient that there is used a combination of the palladium compound and the trialkylphosphine, triphenylphosphine, the trialkyl phosphite, the triaryl phosphite or the phosphorus compound of the general formula (IV). These compounds may be used in the reaction system singly or in the form of a previously prepared complex.

Although not critical, the total amount of the palladium compound and the phosphorus compound(s) added to the reaction system may be chosen in the range of 0.0001 to 0.5 mole, preferably 0.001 to 0.1 mole, per mole of the substituted phenacyl halide derivative of the general formula (II).

The base usable in the present invention may be selected from the group consisting of inorganic bases and organic bases. As said base, there can be exemplified inorganic bases such as sodium carbonate, sodium hydrogencarbonate, potassium carbonate, potassium hydrogencarbonate, sodium hydroxide or potassium hydroxide; and organic bases such as triethylamine, tributylamine, diisopropylethylamine, triisooctylamine, sodium acetate, pyridine, N-methylpyrrolidine, N-methylmorpholine or N-ethylmorpholine.

Although the amount of the base used is preferably an amount necessary for neutralization of the hydrogen halide produced, it may be either larger or smaller than the necessary amount.

As the inert solvent used in the present invention, any inert solvent may be used so long as it does not inhibit the progress of the reaction markedly. There can be used inert solvents such as hexane, benzene, ether, tetrahydrofuran, acetonitrile, dimethyl formamide, dimethylacetamide, dichloromethane, hexamethylphosphoramide, acetone, ethyl acetate, chlorobenzene, fluorobenzene, toluene or dioxane. In addition, the alcohol of the general formula (III) can be used also as a solvent for reaction.

In the present invention, the reaction can be carried out at atmospheric pressure or under pressure. The carbon monoxide pressure is properly chosen in the range of 98.1 to 9810 kPa (1 to 100 atmospheres), and is preferably in the range of 490.5 to 4905 kPa (5 to 50 atmospheres).

The reaction temperature is usually in the range of room temperature to 200°C, preferably room temperature to 150°C.

Any conventional reactor may be used in the present invention. When the reaction is carried out under pressure, any reactor may be used so long as it can withstand the reaction pressure, and a reactor made of metal or glass can usually be used.

Although the reaction time is varied depending on the degree of the reaction and the reaction temperature, it may be chosen in the range of several minutes to 48 hours.

For accelerating the progress of the reaction, the reaction can be carried out by adding a phase transfer catalyst represented by the following general formula: 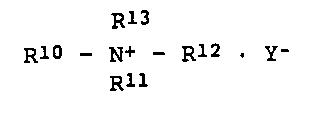 [wherein R¹⁰, R¹¹, R¹² and R¹³, which may be the same or different, are linear C₁₋₆alkyl groups, substituted or unsubstituted phenyl groups, substituted or unsubstituted benzyl groups, or allyl groups, and Y⁻ is a chlorine ion (Cl⁻), fluorine ion (F⁻), bromine ion (Br⁻), iodine ion (I⁻), hydroxide ion (OH⁻), acetate ion (CH₃CO⁻), sulfate ion (1/2 SO₄²⁻, HSO₄⁻), nitrate ion (NO₃⁻), or phosphate ion (H₂PO₄⁻)]. The amount of the phase transfer catalyst added may be chosen in the range of 0.01 to 1 mole per mole of the substituted phenacyl halide derivative of the general formula (II).

Typical examples of the phase transfer catalyst of the above general formula are triethylbenzylammonium chloride and n-cetyltrimethylammonium bromide. But, the phase transfer catalyst is not limited to them.

After completion of the reaction, the desired compound is isolated from the reaction system containing the desired compound by a conventional method and is purified if necessary, whereby the desired compound can be produced.

### Example 1

### Production of ethyl (2,4-dichlorobenzoyl)-acetate

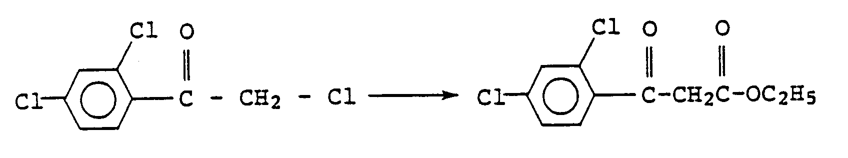

In a 100-ml stainless-steel autoclave were placed 0.2 g (0.90 mmole) of 2,4-dichlorophenacyl chloride, 12.6 mg (0.018 mmole) of dichlorobis(triphenylphosphine)palladium, 23.4 mg (0.09 mmole) of triphenylphosphine, 61.8 mg (0.45 mmole) of potassium carbonate, 0.5 ml of ethanol and 10 ml of toluene. The air in the reaction system was replaced three times with carbon monoxide (981 kPa(10 kg/cm²)), after which the pressure was increased to 2940 kPa (30 kg/cm²) and the reaction was carried out at 80°C for 4 hours.

After completion of the reaction, the reaction system was allowed to cool and the reaction mixture was filtered. The desired compound was extracted from the filtrate with ethyl acetate, and the extracted solution was washed with an aqueous sodium chloride solution, dried over magnesium sulfate, and then concentrated under reduced pressure to obtain 0.2 g of yellow oil.
Physical property:
   - ¹H-NMR: [TMS/CDCl₃, δ values (ppm)]
   1.26 (t, 3H, J=6Hz), 4.01 (2H), 4.19 (q, 2H, J=6Hz), 7.16-7.80(3H, m)
Yield: 90.9%.

### Example 2

### Production of ethyl (5-cyanomethoxy-4-chloro-2-fluorobenzoyl)acetate

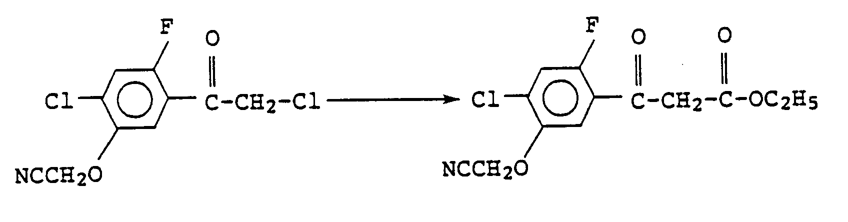

In a 100-ml stainless-steel autoclave were placed 0.1 g (0.38 mmole) of 4-chloro-5-cyanomethoxy-2-fluorophenacyl chloride, 13.3 mg (0.019 mmole) of dichlorobis(triphenylphosphine)palladium, 20.0 mg (0.08 mmole) of triphenylphosphine, 62.2 mg (0.45 mmole) of potassium carbonate, 0.2 ml of ethanol and 5 ml of 1,4-dioxane. The air in the reaction system was replaced three times with carbon monoxide (981 kPa(10 kg/cm²)), after which the pressure was increased to 2940 kPa (30 kg/cm²) and the reaction was carried out at 80°C for 4 hours.

After completion of the reaction, the reaction system was allowed to cool and the reaction mixture was filtered. The desired compound was extracted from the filtrate with ethyl acetate, and the extracted solution was washed with an aqueous sodium chloride solution, dried over magnesium sulfate, and then concentrated under reduced pressure to obtain 0.1 g of yellow crystals.
Physical property:
   - ¹H-NMR: [TMS/CDCl₃, δ values (ppm)]
   3.96 (2H), 4.21 (q, 2H, J=6Hz), 1.26 (t, 3H, J=6Hz), 4.87 (2H), 7.12-7.80 (m, 2H).
Yield: 88.5%.

### Example 3

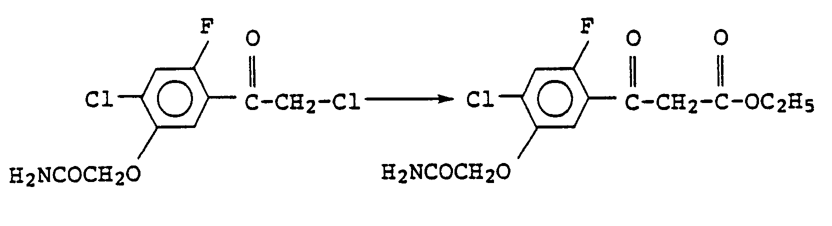

3-1.
In a 100-ml stainless-steel autoclave were placed 1.0 g (3.57 mmoles) of 5-carbamoylmethoxy-4-chloro-2-fluorophenacyl chloride, 2.5 mg (0.0357 mmole) of dichlorobis(triphenylphosphine)palladium, 9.4 mg (0.00357 mmole) of triphenylphosphine, 270.9 mg (1.96 mmoles) of potassium carbonate, 0.5 ml of ethanol and 5 ml of 1,4-dioxane. The air in the reaction system was replaced three times with carbon monoxide (981 kPa(10 kg/cm²)), after which the pressure was increased to 2940 kPa (30 kg/cm²) and the reaction was carried out at 80°C for 4 hours.
After completion of the reaction, the reaction system was allowed to cool and the reaction mixture was filtered. The desired compound was extracted from the filtrate with ethyl acetate, and the extracted solution was washed with an aqueous sodium chloride solution, dried over magnesium sulfate, and then concentrated under reduced pressure to obtain 0.9 g of yellow crystals.
Physical property: m.p. 136 - 138°C.
Yield: 88%.

3-2.
In a 200-ml stainless-steel autoclave were placed 10 g (35.7 mmoles) of 5-carbamoylmethoxy-4-chloro-2-fluorophenacyl chloride, 50 mg (0.07 mmole) of dichlorobis(triphenylphosphine)palladium, 94 mg (0.36 mmole) of triphenylphosphine, 3.3 g (39.3 mmoles) of sodium hydrogencarbonate and 50 ml of ethanol. The air in the reaction system was replaced twice with carbon monoxide (981 kPa(10 kg/cm²)), after which the pressure was increased to 2940 kPa (30 kg/cm²) and the reaction was carried out at 100°C for 4 hours.
After completion of the reaction, the reaction system was cooled to room temperature with ice water, and the pressure was reduced to atmospheric pressure by liberation of carbon monoxide. The reaction mixture thus obtained was concentrated under reduced pressure and its total volume was made to 200 ml by addition of water. The crystals precipitated were collected by filtration, washed with water, and then dried under reduced pressure to obtain 10.57 g of the desired compound.
Yield: 93.2%.

3-3.
Experiments were carried out in the same manner as in 3-1 except for employing various amounts of the catalyst, carbon monoxide pressures, amounts of the solvent, bases and temperatures.
The results obtained are shown in Table 1.

**Table 1**

| No. | Amount of catalyst (mole %) | CO pressure kPa(kg/cm²) | Solvent/reactant (ml) | Base | Temperature (°C) | Yield (%) |
|---|---|---|---|---|---|---|
| 1 | 0.1 | 2940 (30) | EtOH (50) | NaHCO₃ | 80 | 41 |
| 2 | 0.1 | 2940 (30) | EtOH (50) | NaHCO₃ | 90 | 57 |
| 3 | 0.1 | 2940 (30) | EtOH (50) | NaHCO₃ | 100 | 65 |
| 4 | 0.2 | 2940 (30) | EtOH (50) | NaHCO₃ | 100 | 74 |
| 5 | 0.3 | 2940 (30) | EtOH (50) | NaHCO₃ | 100 | 86 |
| 6 | 0.2 | 2940 (50) | EtOH (50) | NaHCO₃ | 100 | 89 |
| 7 | 0.2 | 2940 (30) | EtOH (100) | NaHCO₃ | 100 | 78 |
| 8 | 0.1 | 2940 (30) | EtOH (50) | Na₂CO₃ | 80 | 59 |
| Note: EtOH ethanol | | | | | | |

3-4.
In a 200-ml stainless-steel autoclave were placed 5 g (17.9 mmoles) of 5-carbamoylmethoxy-4-chloro-2-fluorophenacyl chloride, 25 mg (0.036 mmole) of dichlorobis(triphenylphosphine)palladium, 55 mg (0.18 mmole) of triphenyl phosphite, 1.65 g (19.6 mmoles) of sodium hydrogencarbonate and 25 ml of ethanol. The air in the reaction system was replaced twice with carbon monoxide (981 kPa(10 kg/cm²)), after which the pressure was increased to 2940 kPa (30 kg/cm²) and the reaction was carried out at 100°C for 4 hours.
After completion of the reaction, the same treatment as in 3-2 was carried out to obtain 5.37 g of the desired compound.
Yield: 94.5%.

3-6.
Experiments were carried out in the same manner as in 3-2 except for employing various solvents, amounts of the solvents and temperatures.
The results obtained are shown in Table 2.

**Table 2**

| No. | Solvent/reactant (ml) | Base | Tempera-ture (°C) | Yield (%) |
|---|---|---|---|---|
| 9 | EtOH (25) | Na₂CO₃ | 100 | 23 |
| 10 | EtOH (25) | NaOH | 100 | 45 |
| 11 | EtOH (25) | Triethylamine | 100 | 64 |
| 12 | EtOH (25) | Sodium acetate | 100 | 61 |
| 13 | EtOH (25) | Triethylamine | 50 | 82 |
| 14 | EtOH (25) | Sodium acetate | 50 | 87 |
| 15 | EtOH (25) | Sodium acetate | 35 | 77 |
| 16 | EtOH (12.5)+ THF (12.5) | Sodium acetate | 50 | 86 |
| 17 | EtOH (12.5)+ DMF (12.5) | Sodium acetate | 50 | 62 |
| 18 | i-PrOH (25) | Sodium acetate | 50 | 78 |
| 19 | t-BuOH (25) | Sodium acetate | 50 | 85 |
| Note: EtOH ethanol i-PrOH i-propanol t-BuOH t-butanol THF tetrahydrofuran DMF dimethylformamide | | | | |

### Example 4

### Production of ethyl (5-ethoxycarbonylmethoxy-4-chloro-2-fluorobenzoyl)acetate

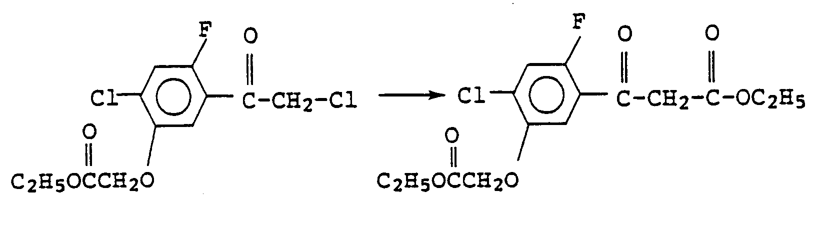

In a 200-ml stainless-steel autoclave were placed 5.0 g (16.2 mmoles) of 5-ethoxycarbonylmethoxy-4-chloro-2-fluorophenacyl chloride, 64 mg (0.03 mmole) of 5% palladium-carbon, 42 mg (0.16 mmole) of triphenylphosphine, 1.49 g (17.7 mmoles) of sodium hydrogencarbonate and 25 ml of ethanol. The air in the reaction system was replaced with carbon monoxide, after which the pressure was increased to 2940 kPa (30 kg/cm²) and the reaction was carried out at 100°C for 6 hours.

After completion of the reaction, the reaction system was allowed to cool and carbon monoxide was liberated. Then, water was added to the reaction mixture and the desired compound was extracted with ethyl acetate, after which the extracted solution was washed with water, dried and then concentrated under reduced pressure to obtain 3.87g of the desired compound.

Physical property: m.p. 24°C. Yield: 69%.

### Example 5

### Production of ethyl (5-N-methylcarbamoyl)-methoxy-4-chloro-2-fluorobenzoyl)acetate

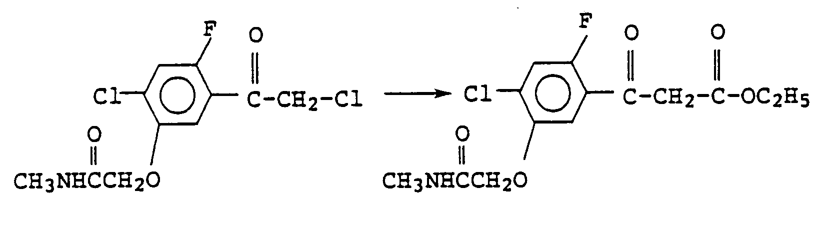

In a 100-ml stainless-steel autoclave were placed 5.0 g (17 mmoles) of 5-(N-methylcarbamoyl)-methoxy-4-chloro-2-fluorophenacyl chloride, 0.15 g (0.07 mmole) of 5% palladium-carbon, 0.04 g (0.15 mmole) of triphenylphosphine, 1.57 g (18.7 mmoles) of sodium hydrogencarbonate and 12.5 ml of ethanol. The air in the reaction system was replaced with carbon monoxide, after which the pressure was increased to 4950 kPa (50 kg/cm²) and the reaction was carried out at 90°C for 6 hours.

After completion of the reaction, the reaction system was allowed to cool and carbon monoxide was liberated. Then, the reaction mixture was filtered and the desired compound was extracted from the filtrate with ethyl acetate. The extracted solution was washed with diluted hydrochloric acid and an aqueous sodium chloride solution, dried over magnesium sulfate, and then distilled under reduced pressure to remove the solvent. The crude product thus obtained was recrystallized from ethyl acetate-n-hexane to obtain 4.6 g of the desired compound as white crystals.
Physical property: m.p. 99.5 - 101.5°C.
Yield: 81%.

From the substituted benzoyl fatty acid derivative obtained by the production process of the present invention, the herbicides disclosed in EPO (Laid-Open) Nos. 0361114 and 0447055 can be produced, for example, by the following process: 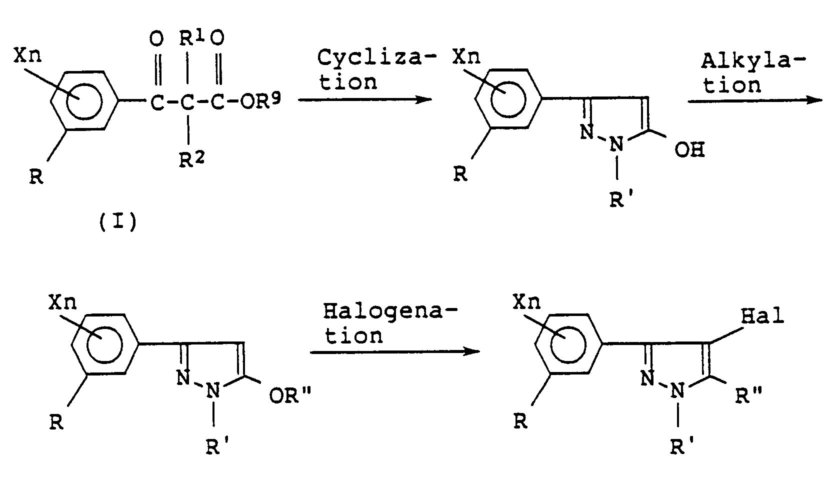 wherein R, R¹, R², R⁹, X, n and Hal are as defined above, and each of R' and R'' is a C₁₋₆alkyl group or a halo-C₁₋₆alkyl group.

## Claims

1. A process for producing a substituted benzoyl fatty acid derivative represented by the general formula (I): 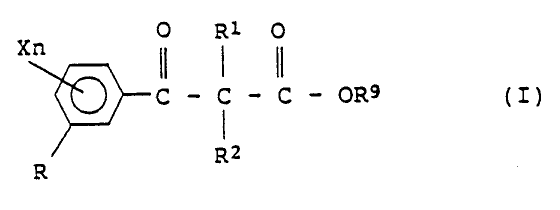 [wherein R is a hydrogen atom or
-O-C(R³)(R⁴)-R⁵
(wherein R³ and R⁴, which may be the same or different, are hydrogen atoms or C₁₋₆alkyl groups, and R⁵ is a cyano group,
-CON(R⁶)R⁷
(wherein R⁶ and R⁷, which may be the same or different, are hydrogen atoms or C₁₋₆alkyl groups) or
-COAR⁸
(wherein R⁸ is a hydrogen atom or a C₁₋₆alkyl group, and A is an oxygen atom or a sulfur atom)), each of R¹ and R² is a hydrogen atom or a C₁₋₆alkyl group, R⁹ is a C₁₋₆alkyl group, an unsubstituted phenyl group, or a substituted phenyl group having one or more substituents selected from the group consisting of halogen atoms, C₁₋₆alkyl groups, halo-C₁₋₆alkyl groups, C₁₋₆alkoxy groups, halo-C₁₋₆alkoxy groups, C₁₋₆alkylthio groups and halo-C₁₋₆alkylthio groups, X's, which may be the same or different, are halogen atoms, and n is an integer of 1 to 3] which comprises reacting a substituted phenacyl halide derivative represented by the general formula (II): 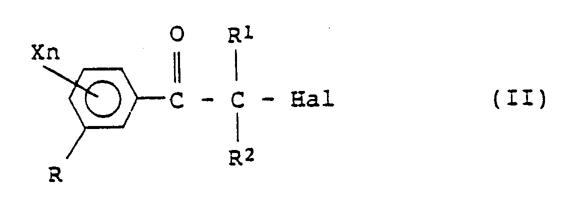 (wherein R, R¹, R², X and n are as defined above, and Hal is a chlorine atom) with a compound represented by the general formula (III):
R⁹-OH
(wherein R⁹ is as defined above) and carbon monoxide in the presence of a base, a palladium compound and at least one phosphorus compound.

2. The process for producing a substituted benzoyl fatty acid derivative according to claim 1, wherein the palladium compound is metallic palladium, or a zerovalent or divalent palladium complex supported on a solid.

3. The process for producing a substituted benzoyl fatty acid derivative according to claim 1 or 2, wherein the palladium compound is palladium-carbon, palladium chloride, palladium acetate, tetrakis(triphenylphosphine)palladium, dichlorobis(benzonitrile)palladium, dichlorobis(acetonitrile)palladium, or dichlorobis(triphenylphosphine )palladium.

4. The process for producing a substituted benzoyl fatty acid derivative according to any of claims 1 to 3 wherein the phosphorus compound is selected from the group consisting of trialkylphosphines, triarylphosphines, trialkyl phosphites, triaryl phosphites and compounds represented by the general formula (IV):
(R')₂-P-Y-P-(R')₂ (IV)
wherein R' is a C₁₋₆alkyl group, an unsubstituted phenyl group, or a substituted phenyl group having one or more substituents selected from the group consisting of halogen atoms, C₁₋₆alkyl groups, halo-C₁₋₆alkyl groups, C₁₋₆alkoxy groups, halo-C₁₋₆alkoxy groups, C₁₋₆alkylthio groups and halo-C₁₋₆alkylthio groups, and Y is a C₁₋₆alkylene group.

5. The process for producing a substituted benzoyl fatty acid derivative according to any of claims 1 to 4 wherein the base is sodium hydrogencarbonate or sodium acetate.

## Patentansprüche

1. Verfahren zur Herstellung eines substituierten Benzoylfensäurederivats, das durch die allgemeine Formel (I) dargestellt wird: 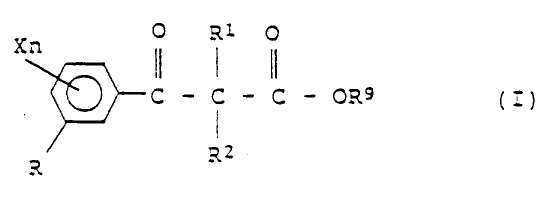 [worin R ein Wasserstoffatom oder
-O-C(R³)(R⁴)-R⁵
darstellt (worin R³ und R⁴, die gleich oder unterschiedlich sein können, Wasserstoffatome von C₁-C₆-Alkylgruppen sind und R⁵ eine Cyangruppe,
-CON(R⁶)R⁷
(worin R⁶ und R⁷, die gleich oder unterschiedlich sein können, Wasserstoffatome oder C₁-C₆-Alkylgruppen sind) oder
-COAR⁸
(worin R⁸ ein Wasserstoffatom oder eine C₁-C₆-Alkylgruppe ist und A ein Sauerstoffatom oder ein Schwefelatom ist) ist), jeweils R¹ und R² ein Wasserstoffatom oder eine C₁-C₆-Alkylgruppe sind, R⁹ eine C₁-C₆-Alkylgruppe, eine nichtsubstituierte Phenylgruppe oder eine substituierte Phenylgruppe mit mindestens einem Substituenten, ausgewählt aus der Gruppe, bestehend aus Halogenatomen, C₁-C₆-Alkylgruppen, Halo-C₁-C₆-Alkylgruppen, C₁-C₆-Alkoxygruppen, Halo-C₁-C₆-Alkoxygruppen, C₁-C₆-Alkylthiogruppen und Halo-C₁-C₆-Alkylthiogruppen ist, X's, die gleich oder unterschiedlich sein können, Halogenatome sind und n eine ganze Zahl von 1 bis 3 ist], umfassend das Reagieren eines substituierten Phenacylhalogenidderivats, das durch die allgemeine Formel (II) dargestellt wird: 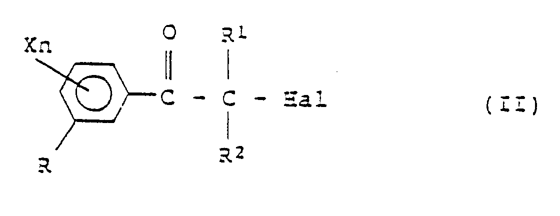 (worin R, R¹, R², X und n wie vorstehend definiert werden und Hal ein Chloratom ist) mit einer Verbindung, die durch die allgemeine Formel (III) dargestellt wird:
R⁹-OH
(worin R⁹ wie vorstehend definiert wird) und Kohlenmonoxid in Gegenwart einer Base, einer Palladiumverbindung und wenigstens einer Phosphorverbindung.

2. Verfahren zur Herstellung eines substituierten Benzoylfettsäurederivats nach Anspruch 1, worin die Palladiumverbindung metallisches Palladium oder ein nullwertiger oder zweiwertiger Palladiumkomplex, der auf einem Feststoff getragen wird, ist.

3. Verfahren zur Herstellung eines substituierten Benzolyfettsäurederivats nach Anspruch 1 oder 2, worin die Palladiumverbindung Palladium-Kohlenstoff, Palladiumchlorid, Palladiumacetat, Tetrakis(triphenylphosphin)palladium, Dichlorbis(benzonitril)palladium, Dichlorbis(acetonitril)palladium oder Dichlorbis(triphenylphosphin)palladium ist.

4. Verfahren zur Herstellung eines substituierten Benzoylfettsäurederivats nach einem der Ansprüche 1 bis 3, worin die Phosphorverbindung ausgewählt wird aus der Gruppe, bestehend aus Trialkylphosphinen, Triarylphosphinen, Trialkylphosphiten, Triarylphosphiten und Verbindungen, die durch die allgemeine Formel (IV) dargestellt werden:
(R')₂-P-Y-P-(R')₂ (IV)
worin R' eine C₁-C₆-Alkylgruppe, eine nichtsubstituierte Phenylgruppe oder eine substituierte Phenylgruppe mit mindestens einem Substituenten, ausgewählt aus der Gruppe, bestehend aus Halogenatomen, C₁-C₆-Alkylgruppen, Halo-C₁-C₆-Alkylgruppen, C₁-C₆-Alkoxygruppen, Halo-C₁-C₆-Alkoxygruppen, C₁-C₆-Alkylthiogruppen und Halo-C₁-C₆-Alkylthiogruppen ist, und Y eine C₁-C₆-Alkylengruppe ist.

5. Verfahren zur Herstellung eines substituierten Benzoylfettsäurederivats nach einem der Ansprüche 1 bis 4, worin die Base Natriumhydrogencarbonat oder Natriumacetat ist.

## Revendications

1. Procédé destiné à produire un dérivé d'acide gras benzoyle substitué représenté par la formule générale (I) : 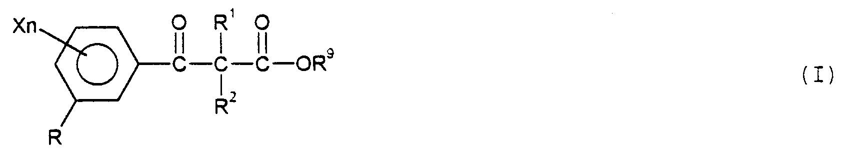 [dans laquelle R est un atome d'hydrogène ou
-O-C(R³)(R⁴)-R⁵
(où R³ et R⁴, qui peuvent être identiques ou différents, sont des atomes d'hydrogène ou des groupes alkyle en C₁ à C₆, et R⁵ est un groupe cyano,
-CON(R⁶)R⁷
(où R⁶ et R⁷, qui peuvent être identiques ou différents, sont des atomes d'hydrogène ou des groupes alkyle en C₁ à C₆) ou
-COAR⁸
(où R⁸ est un atome d'hydrogène ou un groupe alkyle en C₁ à C₆, et A est un atome d'oxygène ou un atome de soufre)), chacun de R¹ et R² est un atome d'hydrogène ou un groupe alkyle en C₁ à C₆, R⁹ est un groupe alkyle en C₁ à C₆, un groupe phényle non substitué, ou un groupe phényle substitué ayant un ou plusieurs substituants choisis dans l'ensemble constitué par les atomes d'halogène, les groupes alkyle en C₁ à C₆, les groupes halogénoalkyle en C₁ à C₆, les groupes alcoxy en C₁ à C₆, les groupes halogénoalcoxy en C₁ à C₆, les groupes alkylthio en C₁ à C₆ et les groupes halogénoalkylthio en C₁ à C₆, les X, qui peuvent être identiques ou différents, sont des atomes d'halogène, et n est un entier de 1 à 3], qui comprend la réaction d'un dérivé halogénure de phénacyle substitué représenté par la formule générale (II) : 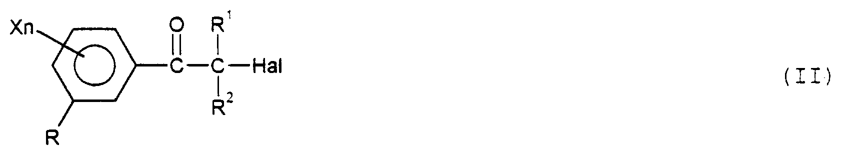 (dans laquelle R, R¹, R², X et n sont tels que définis ci-dessus, et Hal est un atome de chlore) avec un composé représenté par la formule générale (III) :
R⁹-OH
(dans laquelle R⁹ est tel que défini ci-dessus) et du monoxyde de carbone en présence d'une base, d'un composé du palladium et d'au moins un composé du phosphore.

2. Procédé destiné à produire un dérivé d'acide gras benzoyle substitué selon la revendication 1, dans lequel le composé du palladium est du palladium métallique, ou un complexe du palladium de valence zéro ou divalent supporté sur un solide.

3. Procédé destiné à produire un dérivé d'acide gras benzoyle substitué selon la revendication 1 ou 2, dans lequel le composé du palladium est du charbon palladié, du chlorure de palladium, de l'acétate de palladium, du tétrakis(triphénylphosphine)palladium, du dichlorobis(benzonitrile)palladium, du dichlorobis(acétonitrile)palladium, ou du dichlorobis(triphénylphosphine)palladium.

4. Procédé destiné à produire un dérivé d'acide gras benzoyle substitué selon l'une quelconque des revendications 1 à 3, dans lequel le composé du phosphore est choisi dans l'ensemble constitué par les trialkylphosphines, les triarylphosphines, les phosphites de trialkyle, les phosphites de triaryle et les composés représentés par la formule générale (IV) :
(R')₂-P-Y-P-(R')₂ (IV)
dans laquelle R' est un groupe alkyle en C₁ à C₆, un groupe phényle non substitué, ou un groupe phényle substitué ayant un ou plusieurs substituants choisis dans l'ensemble constitué par les atomes d'halogène, les groupes alkyle en C₁ à C₆, les groupes halogénoalkyle en C₁ à C₆, les groupes alcoxy en C₁ à C₆, les groupes halogénoalcoxy en C₁ à C₆, les groupes alkylthio en C₁ à C₆ et les groupes halogénoalkylthio en C₁ à C₆, et Y est un groupe alkylène en C₁ à C₆.

5. Procédé destiné à produire un dérivé d'acide gras benzoyle substitué selon l'une quelconque des revendications 1 à 4, dans lequel la base est l'hydrogénocarbonate de sodium ou l'acétate de sodium.
